# EUROPEAN PATENT SPECIFICATION

(11) **EP 2 429 608 B1**
(45) Date of publication and mention of the grant of the patent: **26.07.2017**
(21) Application number: 11716432.7
(22) Date of filing: 29.04.2011
(51) Int. Cl.: A61M 5/00, B01L 9/00, B65D 77/04, B65D 77/20, B65D 85/42

(54) **PACKAGING STRUCTURE FOR CONTAINERS FOR PHARMACEUTICAL USE**
VERPACKUNGSSTRUKTUR FÜR BEHÄLTER ZUR PHARMAZEUTISCHEN VERWENDUNG
STRUCTURE DE CONDITIONNEMENT POUR RÉCIPIENTS À USAGE PHARMACEUTIQUE

(30) Priority: 30.04.2010 IT MI20100747
(43) Date of publication of application: 21.03.2012
(73) Proprietor: STEVANATO GROUP INTERNATIONAL A. S., 844 03 Bratislava (SK)
(72) Inventor: NICOLETTI, Fabiano, I-30034 Mira (Venezia) (IT)
(74) Representative: Rapisardi, Mariacristina
(86) International application number: PCT/EP2011/056861
(87) International publication number: WO 2011/135085

(56) References cited:
- WO-A1-2009/015862
- US-A- 2 655 283

## Description

The present invention refers to a packaging structure for containers for pharmaceutical use.

A packaging structure for containers for pharmaceutical use is known, comprising a tray supporting at its inside a support plane having a plurality of housing holes in which the containers are positioned with a vertical orientation.

Some containers like the syringes can be held in position by means of a simple rest of their flange on the peripheral edge of the housing holes.

Other containers which do not have protruding parts need suitable elements, permitting to maintain the same inside the housing holes..

Packaging systems for containers of this kind are known for example from the international patent application n. WO2009/015862.

The known packaging structures have a limited flexibility of use, because they are not suitable generally to be used efficiently and universally for packaging different sets of containers in which the containers of a set differ from those of another set for shape and/or size, generally for the height, and in case they are suitable for such an use, they are not able to permit in any case the automatic management of the various sets of containers, by means of processing machines intended for their handling, filling and eventual closure.

The technical scope of the present invention is therefore that to realize a packaging structure for containers for a pharmaceutical use, which permits to eliminate the technical drawbacks lamented in the known technique.

In the field of this technical scope, an aim of the invention is that to realize a packaging structure for containers for pharmaceutical use, which can be universally used to package sets of containers in which each set differs from the other for the height of the containers, and at the same time permits the automatic management of the specific set of containers, used from time to time.

Another scope of the invention is that to realize a packaging structure for containers for pharmaceutical use able to held firmly in position the containers, in a prefixed position in order to protect them from accidental breakings and in order to manage the same in an automatic way with a processing machine.

Another scope of the invention is that to realize a packaging structure for containers for pharmaceutical use, which guarantees the sterilization of the product, maintaining its sterility, its quality properties, the evidence of the integrity of the product, the identification and the traceability of the product, the transferring of the product, without compromising the previously listed properties. The technical scope, and also these and other scopes according to the present invention are reached by realizing a packaging structure for containers for pharmaceutical use accoring to appended claim 1. Preferably said recesses are positioned along the edge of said support plane and said protrusions are positioned along the inner surface of the lateral walls of said tray, and vice versa.

Preferably said support plane has a first engaging position at a first distance from the base of said tray when said protrusions are engaged in said recesses and the edge of the support plane rests on an inner peripheral shoulder of the lateral walls of said tray, and a second engaging position at a second distance from the base of said greater tray of said first distance, when said recesses are disengaged from said protrusions on which the edge of said support plane rests.

Preferably said protrusions and said recesses are positioned such that said first and second engaging positions are obtained by a 180° rotation of said support plane with respect to said tray, around its own central axis, orthogonal to the resting plane of the tray.

Preferably said seats comprise circular through holes, with a matrix disposition. Preferably said support plane has, at each circular through hole, a system for resting a container.

Preferably said resting system has a resting portion, contained in the cylindrical space, comprised between the cylindrical gene rating elements of said circular hole and it is configured in order to liberate a diametric plane of said cylindrical space.

Preferably the free diametric spaces, corresponding to the holes of each rows of holes are mutually coplanar.

Preferably to the free diametric coplanar planes is associated a free total volume, so to permit the introduction from below of a rising element, simultaneous of one or more containers.

Preferably said closing element is an exfoliative sheet from the tray.

Preferably said closing element is in a selective material permeable to a sterilization gas of the contents of the tray.

Preferably the tray has a protective envelope to be transferred in a controlled zone. Preferably to the tray an identification and traceability element is associated.

Further characteristics and advantages of the invention will be clearer from the description of a preferred but not exclusive embodiment of the packaging structure for containers for pharmaceutical use, according to the founding, here shown for indicative and non limiting way in the annexed drawings, in which:

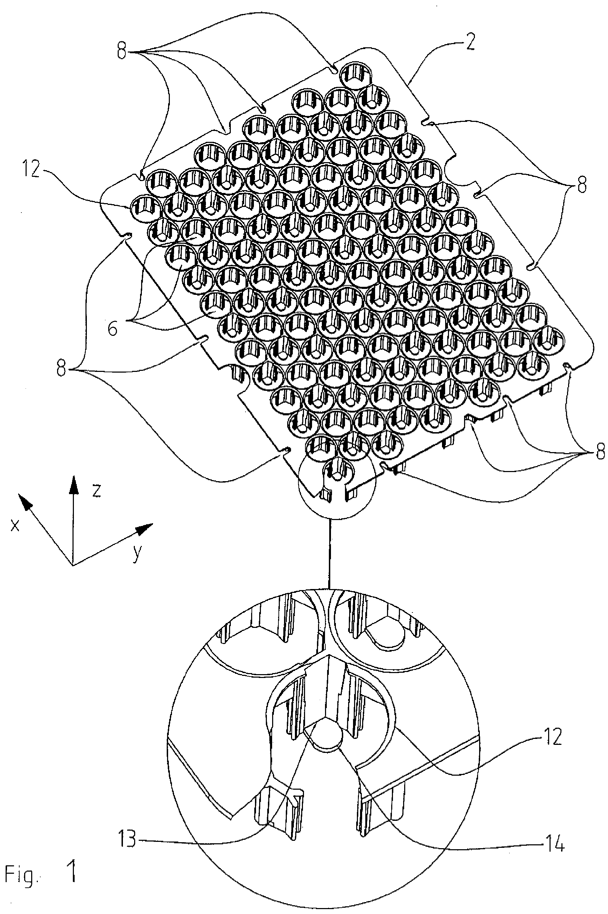

Figure 1 1a shows a perspective view of the support plane free from containers, and one enlarged detail of the same;

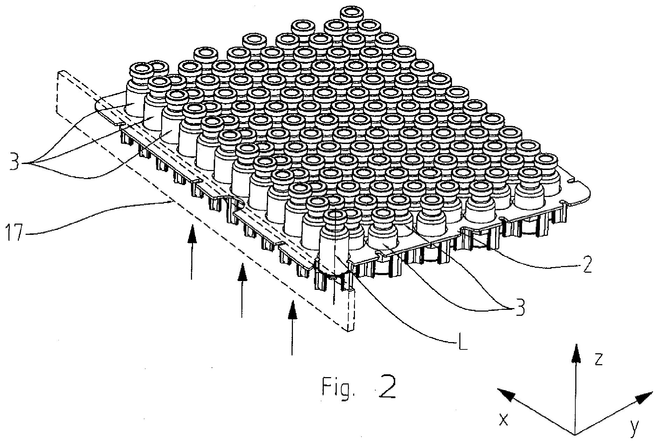

Figure 2 shows a perspective view of a portion of the support plane with the housed containers and a handling beam of the rows of containers;

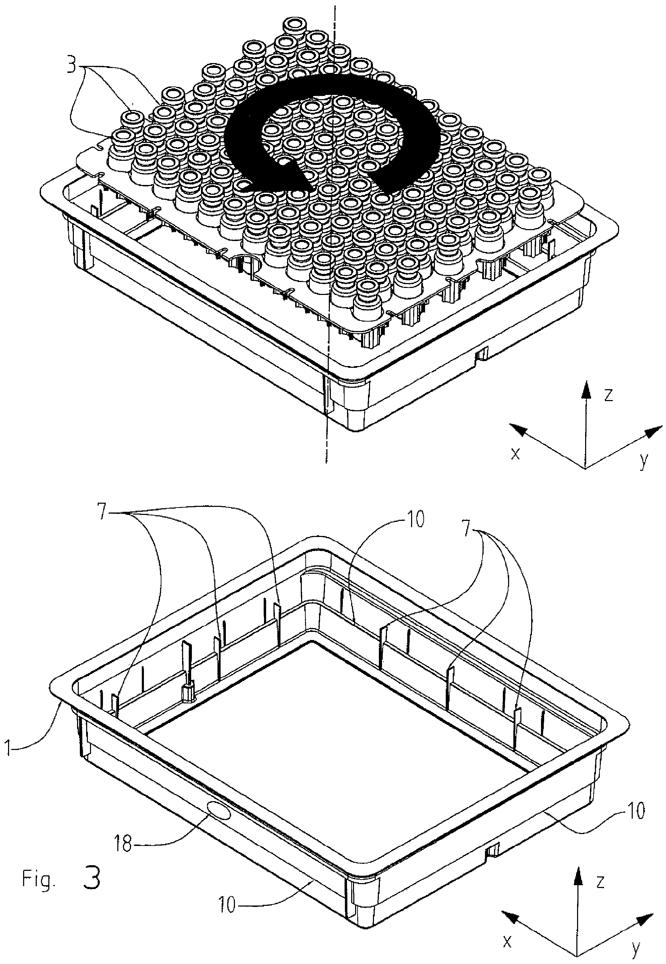

Figure 3 shows a perspective view of the tray with a set of identical containers with a certain height;
Figure 4a shows a top side view of the tray with the support plane in the first engaging position in which it houses a set of containers having a certain height;
Figure 4b shows a top side view of the tray with the support plane in the second engaging position in which it houses a set of containers with a height lower than those shown in figure 4a;
Figure 5 shows a plan view of the tray free from containers with the support plane in its first engaging position;
Figure 6 shows a top side view of the tray, sectioned along the line 6-6 in figure 5;
Figure 7 shows a plan view of the tray with the support plane in its second engaging position;
Figure 8 shows a top side view of the tray, sectioned along the line 8-8 in figure 7;

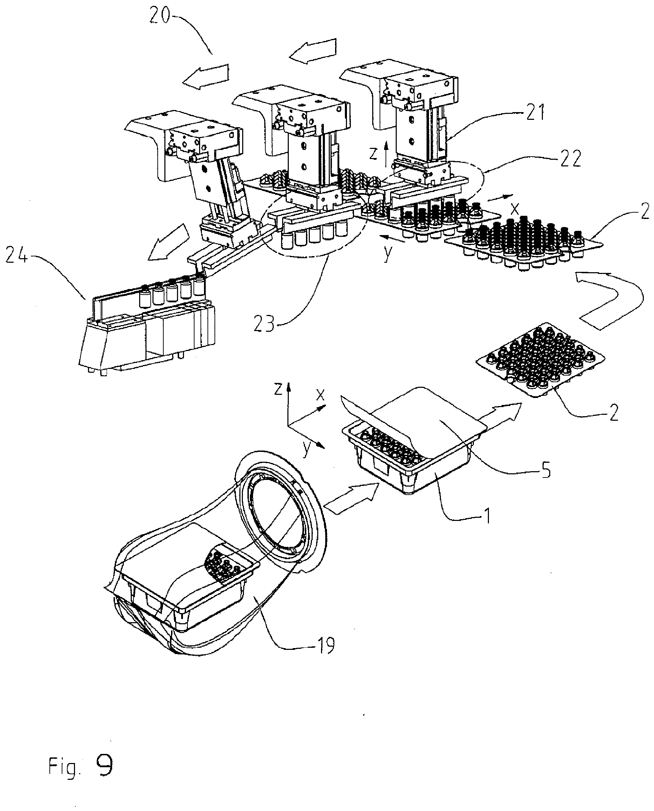

Figure 9 shows a possible automatic handling layout of the containers, with a processing machine;
Figures 10 and 11 show a different processing machine, shown with and without an insulator for clarity aims, suitable to process the containers according to the present invention.

With reference to the cited figures, a packaging structure for containers for pharmaceutical use is shown, for example glass or plastic bottles.

The packaging structure comprises a tray 1 having an open side for introducing and extracting a support plane 2 of the containers 3, 3' which can be positioned parallel with the resting plane 4 of the tray 1, and with a closing element 5 of the open side of the tray 1.

The support plane 2 has a spatially prefixed distribution of seats 6 in which the containers 3, 3' can be positioned at a mutual distance with their longitudinal axis L, L' orthogonal to the resting plane 4 of the tray 1.

The support plane 2 has a quadrangular shape with pairs of opposed parallel edges 2a, 2a, and 2b, 2b.

Analogously the tray 1 has a quadrangular shape with a base 9 provided parallel to the resting plane 4 and pairs of opposed lateral walls 1a, 1a and 1b, 1b.

The packaging structure comprises diversification means of the engaging configuration between the support plane 2 and the tray 1 able to modify the engaging position of the support plane 2 in the tray 1 along the direction Z orthogonal to the resting plane 4 of the tray 1 in order to maintain unchanged the points inside the tray 1 in which an end 3a, 3a' of the containers 3, 3' is positioned, with the varying of the height of the containers 3, 3' of the container set which is used from time to time.

The diversification means are studied in order to guarantee the drive and the alignment of the containers 3, 3' during the various phases of the process.

It is clear that the height of the containers 3, 3' corresponds to their size in the direction of their longitudinal axis L, L'.

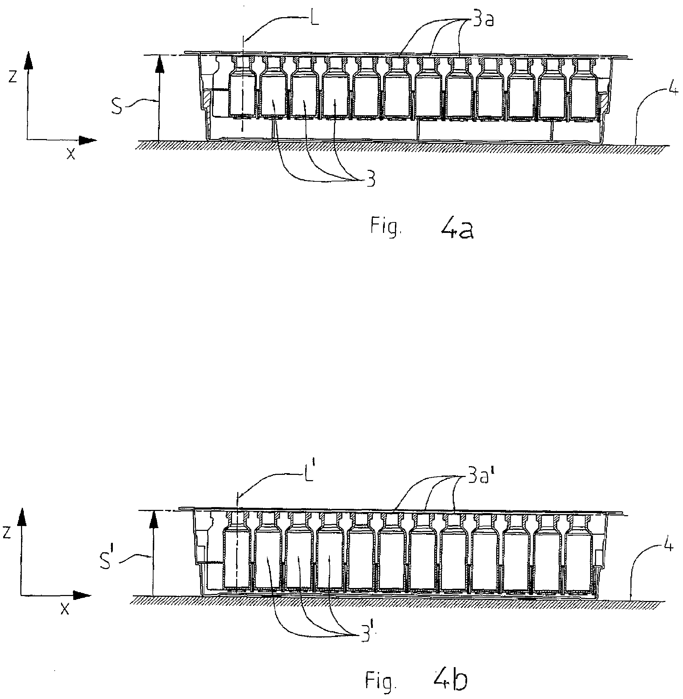

From the comparison between Figures 4a and 4b it is noted that the distance S of the end 3a of a container 3 from the base 9 of the tray 1 corresponds to the distance S' of the end 3 a' of a container 3' from the base 9 of the tray 1.

The diversification means comprise protrusions 7 provided with recesses 8 in a conjugated form.

The support plane 2 has a first engaging position at a first distance from the base 9 of the tray 1 when the protrusions 7 are engaged in the recesses 8 and the edge of the support plane 2 rests on inner peripheral shoulder 10 of the lateral walls 1a, 1a and 1b, 1b of the tray 1, and a second engaging position at a second distance from the bottom 9 of the tray 1 greater than the first distance, when the recesses 8 are disengaged from the protrusions 7 on which the edge of the support plane 2 is resting.

The recesses 8 are positioned along the pairs of opposed parallel edges 2a, 2a and 2b, 2b of the support plane 2 whereas the protrusions 7 are positioned along the inner surface of the lateral walls 1a, 1a and 1b, 1b of the tray 1.

In particular, a first lateral wall 1a of the tray 1 has three protrusions 7 and a second lateral wall 1a has three protrusions 7 offset from those of the first lateral wall 1a along the common direction of the first and second lateral walls 1a.

A first lateral wall 1b of the tray 1 has four protrusions 7 and a second lateral wall 1b has four protrusions 7 offset from those of the first lateral wall 1b along the common direction of the first and second lateral wall 1b.

A first edge 2a of the support plane 2 has four recesses 8 and a second edge 2a of the support plane 2 has three recesses 8 offset from those of the first edge 2a along the common direction of the edges 2a.

A first edge 2b of the support plane 2 has four recesses 8 and a second edge 2b of the support plane 2 has four recesses 8 offset from those of the first edge 2b along the common direction of the edges 2b.

The protrusions 7 and the recesses 8 are positioned so that the passage between the first and second engaging position is conferred by rotation of 180° of the support plane 2 with respect to the tray 1 around its central axis T orthogonal to the resting plane 4 of the tray 1.

In practice, when the protrusions 7 are aligned with the recesses 8, these latter can slide along the protrusions 7 which extend themselves again longitudinally along the direction Z orthogonal to the resting plane 4, and the support plane 2 can move towards the base 9 of the tray 1 until it rests on the shoulder 10.

In the other hand, when the protrusions 7 are not aligned from the recesses 8, the protrusions 7 rest themselves on the support plane 2.

Let us consider now a Cartesian reference system in which X and Y correspond with the axis XY plane, corresponding with the resting plane 4 in which the base 9 of the tray 1is housed and Z, as aforesaid, is orthogonal to the resting plane 4.

In order to guarantee that the points inside the tray 1 remain unchanged where an end 3a, 3a' of the containers 3, 3' at the varying of the height of the containers 3, 3' of the set of containers (size along the axis Z of the containers 3, 3' when associated to their seats 6), used from time to time, also the distribution of the seats 6 in the plane XY must be such to reproduce itself in the same way by rotation of 180° of the support plane 2 with respect to the tray 1 around its central axis T parallel to the Cartesian axis Z.

The seats 6 comprise circular through holes 12 with a matrix arrangement in directions X e Y.

At each hole 12 a system for resting a container 3, 3', in particular a foot 13.

The foot 13 has a resting portion 14 contained in the cylindrical space 15 comprised among the generation lines of the hole 12 parallel with the axis Z. Advantageously the portion 14 of the foot 13 is configured in order to free a diameter plane 16 of the cylindrical space 15.

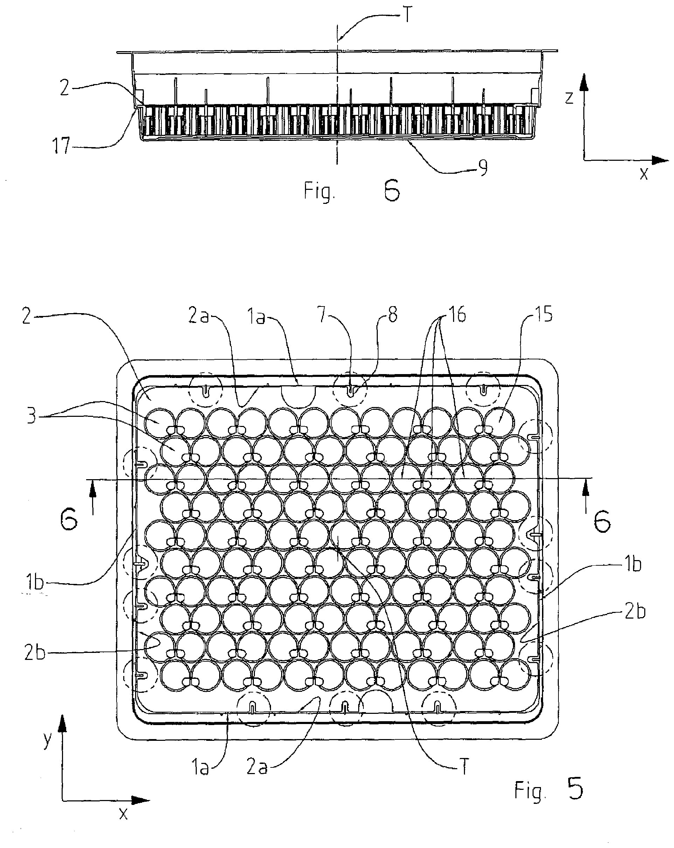

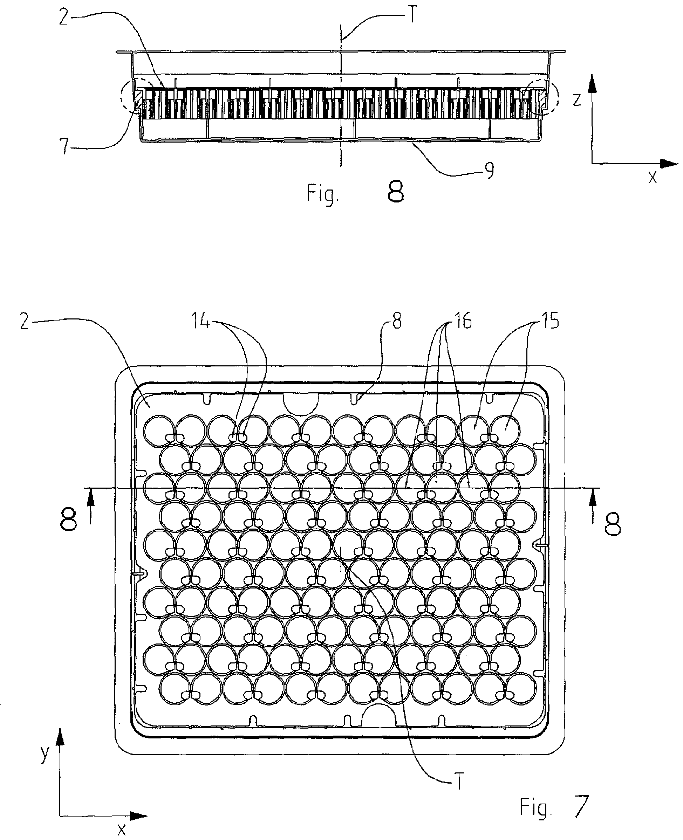

More precisely, the diameter planes 16 free from the cylindrical spaces 15 corresponding to holes 12 of each row of holes 12 are mutually coplanar. In the figures 5 and 7 they occupy parallel planes with respect to the axis XZ. This permit the automatic handling of the containers 3, 3' in rows, by means of a beam 17 positioned under the support plane 2, when this is extracted from the tray 1. The beam 17 is placed longitudinally and parallel with the axis X under a row of containers 3, 3' developing in the direction X, and it can be handled in direction Z. The closing element 5 is a sheet free from foils to the tray 1 realized in a selectively permeable material with a sterilized process contained in the tray 1.

To the tray 1 an identification and traceability element 18 is associated and it is for example a code which can be decoded by means of RFID technology.

The tray 1 can have a housing 19 for protecting its transfer in a controller zone (for example in an area where the filling of the containers occurs)

The protective housing 19 can be closed or open and in particular it can be provided or not with a rapid transfer gate. Each protective housing 19 can contain one or more stacked trays 1, and it is formed, at least for one portion, by a sheet realized of a selectively permeable material with a sterilization process of the contents of the tray 1.

The packaging structure is used 1 for feeding directly and in an automatic way the containers 3, 3' with a spatially prefixed to a processing machine 20 for their handling.

The processing machine 20 can operate in the same way both in presence of containers 3, and of containers 3' because they, even if they have a different height, are directed to the processing machine 20 their end 3a, 3a' in their positions (see a comparison between figures 4a and 4b).

The processing machine 20 comprises a robot arm 21 having a head 22 for the handle of the containers 3, 3'.

The robot arm 21 has a horizontal translation axis in the X direction, a vertical translation axis in the Z direction and a rotary axis according the rotary axis Y. The handling head 22 has a rectilinear open groove 23 for handling a row of containers 3, 3'.

The support plane 2, after having been extracted from the outer housing 19 and free from the closing element 5, is handled towards the handling position by means of the handling head 22 in which the containers 3, 3' are directed, in rows oriented parallel to the axis X.

In particular the handling head 22 is initially external with respect to the shape of the support plane 2 and it has the groove 23 oriented parallel to the axis X.

The handling head 22 performs a translation of the axis X in order to insert the groove 23 in the neck of the row of containers 3, 3' aligned with the groove 23 itself.

In order to facilitate the extraction of the containers 3, 3' is actuated in direction Z the beam 17 which is oriented parallel to the axis X. The beam 17 causes a partial extraction of the row of containers 3, 3' which then must catch the handling head 22.

After having hanged the row of containers 3, 3' the handling head 22 performs a translation of the axis Z in order to rise again the row of containers 3, 3' which is hanged by separating it completely from the support plane 2.

Later on, the handling head 22 performs a translation along the X axis, in order to transport the row of containers 3,3' fixed towards a conveying station 24 at which the handling head 22 rotates around Y axis in order to discharge in the conveyor station 24 the fixed row of containers 3, 3'.

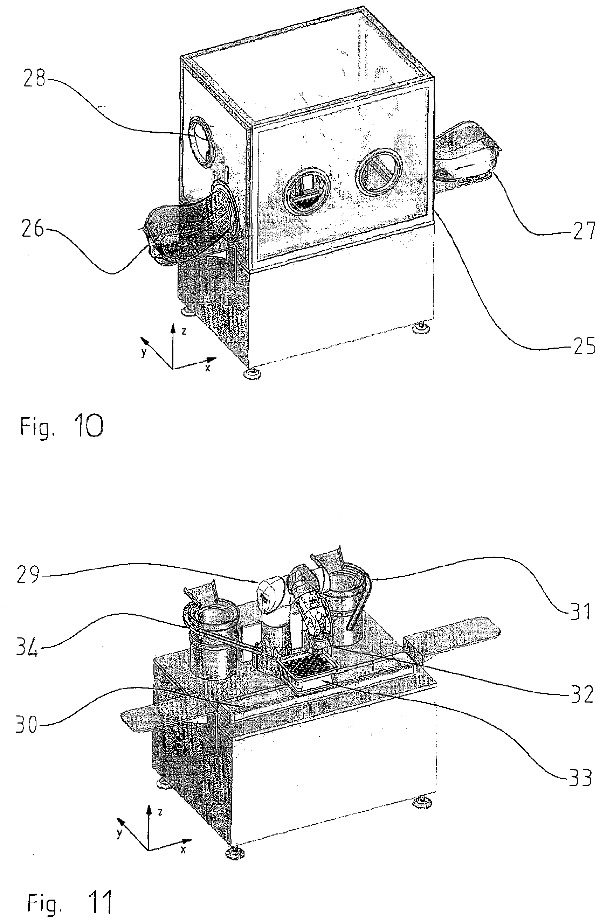

With reference to Figures figure 10 and 11, the processing machine comprises an insulating cover 25 with a vertical, laminar airflow downwards, in order to maintain clean the inside of the containers 3, 3'.

The cover 25 has an inlet rapid transferring gate 26 of the containers 3, 3' and an outlet rapid transferring gate 27 of the containers 3, 3', besides other gates, of which a gate 28 for example for a handling by means of a glove (not shown)

The processing machine has a translation slid 30 with a movement directed step by step, in order to bring the containers 3, 3' from the inlet gate 26 to the reference position, under an anthropomorphic robot 29 and from there to the outlet gate 27.

At the opposite sides of the robot 29 a first vibration distributor 34, and a second vibration distributor 31 are provided. They contain accessories to apply to the containers 3, 3', for example one with plugs and the other with the ring crimp.

The robot is able to recognize the position of the containers 3, 3', because they have a prefixed spatial position, and it provides, by means of a manipulator 32 before their filling, then at the closure, with a plug taken from one of the distributors and finally to the hermetic closure of the plug by application of the crimp ring.

The packaging structure for containers for pharmaceutical use so conceived is susceptible of various changes and variants, all within the scope of the appended claims; furthermore all the details can be substituted by technical equivalent elements.

For example, the number of engaging positions of the support plane with the tray can be greater than two, the passage among the various engaging configurations can be obtained also with a rotary translation movement of the support plane also different from the shown one, and also the structure of the diversification means.

In practice, the materials used, and also the sizes can be of any kind, according to the needs and to the state of the art.

## Claims

1. A packaging structure for containers (3, 3') for pharmaceutical use, pre-treated, sterile and ready to be used, comprising a tray (1), a support plane (2) of the containers and a closing element (5), said tray (1) having an open side for introducing and extracting a support plane (2) of the containers (3, 3'), and said closing element (5) closing off the open side of the tray (1), said support plane (2) having a spatial prefixed distribution of seats (6) in which said containers (3, 3') can be precisely positioned without mutual contact, **characterized in that** it comprises diversification means (7, 8) of the engaging configuration between said support plane (2) and said tray (1) in order to maintain unchanged the points inside the tray (1) in which an end (3a, 3a') of the containers (3, 3') is placed, with the variation of the height of the containers (3, 3') of the container set associated from time to time to the support plane (2), and **in that** said support plane (2) of the containers (3, 3') is positioned parallel to the resting plane (4) of the tray (1), said containers (3, 3') can be positioned in said seats (6) with their longitudinal axis (L, L') orthogonal to the resting plane (4) of the tray (1), and **in that** said diversification means modify the engaging position of said support plane (2) in said tray (1) along the direction (Z) orthogonal to said resting plane (4) of said tray (2).

2. The packaging structure for containers (3, 3') for pharmaceutical use according to claim 1, **characterized in that** said diversification means comprise protrusions (7) provided with recesses (8) in a conjugated form.

3. The packaging structure for containers (3, 3') for pharmaceutical use according to one or more preceding claims, **characterized in that** said recesses (8) are positioned along the edge (11) of said support plane (2) and said protrusions (7) are positioned along the inner surface of the lateral walls (1a, 1a, 1b, 1b) of said tray (1) or vice versa.

4. The packaging structure for containers (3, 3') for pharmaceutical use according to one or more preceding claims, **characterized in that** said support plane (2) has a first engaging position at a first distance from the base (9) of said tray (1) when said protrusions (7) are engaged in said recesses (8) and the edge of said support plane (2) rests on an inner peripheral shoulder (10) of the lateral walls (1a, 1a, 1b, 1b) of said tray (1), and a second engaging position at a second distance (9) of said tray (1) that is greater than the first distance when said recesses (8) are disengaged from said protrusions (7) on which the edge of said support plane (2) rests.

5. The packaging structure for containers (3, 3') for pharmaceutical use according to one or more preceding claims, **characterized in that** said protrusions (7) and said recesses (8) are positioned so that said first and second engaging positions are obtained by a 180° rotation of said support plane (2) with respect to said tray (1) around its central axis (T) orthogonal to the resting plane (4) of the tray (1).

6. The packaging structure for containers (3, 3') for pharmaceutical use according to one or more preceding claims, **characterized in that** said seats (6) comprise circular through holes (12) with a matrix disposition.

7. The packaging structure for containers (3, 3') for pharmaceutical use, according to the preceding claim, **characterized in that** said support plane (2) has, at each hole (12), a system able to sustain (13) the containers (3, 3').

8. The packaging structure for containers (3, 3') for pharmaceutical use according to the preceding claim, **characterized in that** said system (13) able to sustain the containers (3, 3') has a resting portion (14) contained in the cylindrical space (15) comprised between the cylindrical generators of said holes (12) and it is configured in order to free a diametric plane (16) of said cylindrical space (15).

9. The packaging structure for containers (3, 3') for pharmaceutical according to the preceding claim, **characterized in that** the diametric planes (16) free from the cylindrical spaces (15) corresponding to holes (12) of each row of holes (12) are mutually coplanar.

10. The packaging structure for containers (3, 3') for pharmaceutical use according to the preceding claim, **characterized in that** to the free and coplanar diametric planes (16) a free total volume is associated, in order to permit the introduction from below of a rising element (17) for a simultaneous action on one or more containers (3, 3').

11. The packaging structure for containers (3, 3') for pharmaceutical use according to one or more preceding claims, **characterized in that** said closing element (5) is an exfoliative sheet from the tray(1).

12. The packaging structure for containers (3, 3') for pharmaceutical use according to one or more preceding claims, **characterized in that** said closing element (5) is of a selective material permeable to a sterilization process of the contents of the tray (1).

13. The packaging structure for containers (3, 3') for pharmaceutical use according to one or more preceding claims, **characterized in that** it has a closed protective housing (19), which guarantees the sterility of the tray (1) or a housing having at least one rapid transferring gate for its introduction in a controlled zone.

14. The packaging structure for containers (3, 3') for pharmaceutical use, according to one or more preceding claims, **characterized in that** to the tray (1) an identification and traceability element (18) is associated.

15. Use of a packaging structure for containers (3, 3') for pharmaceutical use according to one or more preceding claims in a process machine for the automatic movement and/or the automatic filling and/or for the automatic closure of the containers (3, 3').

## Patentansprüche

1. Verpackungsstruktur für Behälter (3, 3') zur pharmazeutischen Verwendung, vorbehandelt, steril und einsatzbereit, umfassend ein Tray (1), eine Stützebene (2) für die Behälter und ein Verschließelement (5), wobei das Tray (1) eine offene Seite für das Einführen und Herausnehmen einer Stützebene (2) der Behälter (3, 3') umfasst und das Verschließelement (5) die offene Seite des Trays (1) verschließt, wobei die Stützebene (2) eine räumlich vorgegebene Verteilung von Aufnahmen (6) aufweist, in die die Behälter (3, 3') präzise ohne gegenseitigen Kontakt positioniert werden können, **dadurch gekennzeichnet, dass** sie Diversifizierungsmittel (7, 8) der Eingriffskonfiguration zwischen der Stützebene (2) und dem Tray (1) umfasst, um die Punkte im Tray (1), in die ein Ende (3a, 3a') der Behälter (3, 3') positioniert wird, bei der Veränderung der Höhe der Behälter (3, 3') der Behältergruppe, die jeweils mit der Stützebene (2) assoziiert wird, unverändert aufrechtzuerhalten, und
dadurch, dass die Stützebene (2) der Behälter (3, 3') parallel zur Auflageebene (4) des Trays (1) positioniert ist, wobei die Behälter (3, 3') in den Aufnahmen (6) positioniert werden können, indem ihre Längsachsen (L, L') rechtwinklig zur Auflageebene (4) des Trays (1) angeordnet sind, und dadurch, dass die Diversifizierungsmittel die Eingriffsposition der Stützebene (2) im Tray (1) entlang der Richtung (Z) verändern, die rechtwinklig zur Auflageebene (4) des Trays (2) angeordnet ist.

2. Verpackungsstruktur für Behälter (3, 3') zur pharmazeutischen Verwendung nach Anspruch 1, **dadurch gekennzeichnet, dass** die Diversifizierungsmittel Vorsprünge (7) umfassen, die mit Vertiefungen (8) in einer passenden Form versehen sind.

3. Verpackungsstruktur für Behälter (3, 3') zur pharmazeutischen Verwendung nach einem oder mehreren der vorhergehenden Ansprüche, **dadurch gekennzeichnet, dass** die Vertiefungen (8) entlang der Kante (11) der Stützebene (2) positioniert sind und die Vorsprünge (7) entlang der innenseitigen Oberfläche der Seitenwände (1a, 1a, 1b, 1b) des Trays (1) oder umgekehrt positioniert sind.

4. Verpackungsstruktur für Behälter (3, 3') zur pharmazeutischen Verwendung nach einem oder mehreren der vorhergehenden Ansprüche, **dadurch gekennzeichnet, dass** die Stützebene (2) eine erste Eingriffsposition in einem ersten Abstand von der Basis (9) des Trays (1) aufweist, wenn die Vorsprünge (7) in die Vertiefungen (8) eingreifen und die Kante der Stützebene (2) auf einer innenseitigen Umfangsschulter (10) der Seitenwände (1a, 1a, 1b, 1b) des Trays (1) ruht, und eine zweite Eingriffsposition in einem zweiten Abstand (9) des Trays (1), der größer ist als der erste Abstand, wenn die Vertiefungen (8) aus den Vorsprüngen (7) gelöst sind, auf denen die Kante der Stützebene (2) ruht.

5. Verpackungsstruktur für Behälter (3, 3') zur pharmazeutischen Verwendung nach einem oder mehreren der vorhergehenden Ansprüche, **dadurch gekennzeichnet, dass** die Vorsprünge (7) und die Vertiefungen (8) so positioniert sind, dass die erste und die zweite Eingriffsposition durch eine 180°-Drehung der Stützebene (2) zum Tray (1) rund um deren mittige Achse (T) rechtwinklig zur Auflageebene (4) des Trays (1) erzielt werden.

6. Verpackungsstruktur für Behälter (3, 3') zur pharmazeutischen Verwendung nach einem oder mehreren der vorhergehenden Ansprüche, **dadurch gekennzeichnet, dass** die Aufnahmen (6) kreisförmige Durchführungslöcher (12) mit einer Matrixanordnung umfassen.

7. Verpackungsstruktur für Behälter (3, 3') zur pharmazeutischen Verwendung nach dem vorhergehenden Anspruch, **dadurch gekennzeichnet, dass** die Stützebene (2) an jedem Loch (12) ein System aufweist, das in der Lage ist, die Behälter (3, 3') zu halten (13).

8. Verpackungsstruktur für Behälter (3, 3') zur pharmazeutischen Verwendung nach dem vorhergehenden Anspruch, **dadurch gekennzeichnet, dass** das System (13), das in der Lage ist, die Behälter (3, 3') zu halten, einen Auflageabschnitt (14) aufweist, der im zylindrischen Bereich (15) zwischen den zylindrischen Erzeugern der Löcher (12) enthalten und ausgelegt ist, um eine diametrische Ebene (16) vom zylindrischen Bereich (15) zu befreien.

9. Verpackungsstruktur für Behälter (3, 3') zur pharmazeutischen Verwendung nach dem vorhergehenden Anspruch, **dadurch gekennzeichnet, dass** die diametrischen Ebenen (16) frei von den zylindrischen Bereichen (15) entsprechend Löchern (12) einer jede Reihe von Löchern (12) komplanar zueinander angeordnet sind.

10. Verpackungsstruktur für Behälter (3, 3') zur pharmazeutischen Verwendung nach dem vorhergehenden Anspruch, **dadurch gekennzeichnet, dass** mit den freien und komplanaren diametrischen Ebenen (16) ein freies Gesamtvolumen assoziiert ist, um das Einführen eines steigenden Elements (17) von unten für die gleichzeitige Wirkung auf einem oder mehreren Behältern (3, 3') zu erlauben.

11. Verpackungsstruktur für Behälter (3, 3') zur pharmazeutischen Verwendung nach einem oder mehreren der vorhergehenden Ansprüche, **dadurch gekennzeichnet, dass** das Verschließelement (5) ein sich vom Tray (1) ablösendes Blatt ist.

12. Verpackungsstruktur für Behälter (3, 3') zur pharmazeutischen Verwendung nach einem oder mehreren der vorhergehenden Ansprüche, **dadurch gekennzeichnet, dass** das Verschließelement (5) aus einem selektiven Material besteht, das für einen Sterilisierungsprozess des Inhalts des Trays (1) durchlässig ist.

13. Verpackungsstruktur für Behälter (3, 3') zur pharmazeutischen Verwendung nach einem oder mehreren der vorhergehenden Ansprüche, **dadurch gekennzeichnet, dass** sie ein abgeschlossenes Schutzgehäuse (19) aufweist, das die Sterilität des Trays (1) garantiert, oder ein Gehäuse, das mindestens eine Schnellübergabeschleuse für dessen Einführung in einen kontrollierten Bereich aufweist.

14. Verpackungsstruktur für Behälter (3, 3') zur pharmazeutischen Verwendung nach einem oder mehreren der vorhergehenden Ansprüche, **dadurch gekennzeichnet, dass** mit dem Tray (1) ein Identifizierungs- und Rückverfolgungselement (18) assoziiert ist.

15. Verwendung einer Verpackungsstruktur für Behälter (3, 3') zur pharmazeutischen Verwendung nach einem oder mehreren der vorhergehenden in einer Prozessmaschine für das automatische Bewegen und/oder das automatische Befüllen und/oder das automatische Verschließen der Behälter (3, 3').

## Revendications

1. Structure de conditionnement pour récipients (3, 3') à usage pharmaceutique pré-traités, stériles et prêts à être utilisés, comprenant un plateau (1), un plan de support (2) des récipients et un élément de fermeture (5), ledit plateau (1) comportant un côté ouvert pour introduire et extraire un plan de support (2) des récipients (3, 3'), et ledit élément de fermeture (5) refermant le côté ouvert du plateau (1), ledit plan de support (2) comportant une répartition spatiale prédéfinie de sièges (6) dans lesquels lesdits récipients (3, 3') peuvent être positionés précisément sans contact mutuel, **caractérisée en ce qu'**elle comprend des moyens de diversification (7, 8) de la configuration d'engagement entre ledit plan de support (2) et ledit plateau (1) afin de maintenir inchangés les points à l'intérieur du plateau (1) dans lequel une extrémité (3a, 3a') des récipients (3, 3') est placée, avec la variation de la hauteur des récipients (3, 3') de l'ensemble des récipients associé de temps à autre au plan de support (2), et **en ce que** ledit plan de support (2) des récipients (3, 3') est positionné de façon parallèle au plan d'appui (4) du plateau (1), lesdits récipients (3, 3') peuvent être positionnés dans lesdits sièges (6) avec leurs axes longitudinaux (L, L') orthogonaux au plan d'appui (4) du plateau (1), et **en ce que** lesdits moyens de diversification modifient la position d'engagement dudit plan de support (2) dans ledit plateau (1) le long de la direction (Z) orthogonale au dit plan d'appui (4) dudit plateau (2).

2. Structure de conditionnement pour récipients (3, 3') à usage pharmaceutique selon la revendication 1, **caractérisée en ce que** lesdits moyens de diversification comprennent des saillies (7) pourvues de renfoncements (8) sous une forme conjuguée.

3. Structure de conditionnement pour récipients (3, 3') à usage pharmaceutique selon une ou plusieurs des revendications précédentes, **caractérisée en ce que** lesdits renfoncements (8) sont positionnés le long du bord (11) dudit plan de support (2) et lesdites saillies (7) sont positionnées le long de la surface intérieure des cloisons latérales (1a, 1a, 1b, 1b) dudit plateau (1) ou vice versa.

4. Structure de conditionnement pour récipients (3, 3') à usage pharmaceutique selon une ou plusieurs des revendications précédentes, **caractérisée en ce que** ledit plan de support (2) comporte une première position d'engagement à une première distance de la base (9) dudit plateau (1) lorsque lesdites saillies (7) sont engagées dans lesdits renfoncements (8) et le bord dudit plan de support (2) repose sur un épaulement périphérique intérieur (10) des parois latérales (1a, 1a, 1b, 1b) dudit plateau (1), et une seconde position d'engagement à une seconde distance (9) dudit plateau (1) étant supérieure à la première distance lorsque lesdits renfoncements (8) sont désengagés desdites saillies (7) sur lesquelles le bord dudit plan de support (2) repose.

5. Structure de conditionnement pour récipients (3, 3') à usage pharmaceutique selon une ou plusieurs des revendications précédentes, **caractérisée en ce que** lesdites saillies (7) et lesdits renfoncements (8) sont positionnés de manière à ce que lesdites première et seconde positions d'engagement sont obtenues par une rotation de 180° dudit plan de support (2) par rapport au dit plateau (1) autour de son axe central (T) orthogonal au plan d'appui (4) du plateau (1).

6. Structure de conditionnement pour récipients (3, 3') à usage pharmaceutique selon une ou plusieurs des revendications précédentes, **caractérisée en ce que** lesdits sièges (6) comprennent des trous passants circulaires (12) avec une disposition en matrice.

7. Structure de conditionnement pour récipients (3, 3') à usage pharmaceutique selon la revendication précédente, **caractérisée en ce que** ledit plan de support (2) comporte, en correspondance de chaque orifice (12), un système pouvant supporter (13) les récipients (3, 3').

8. Structure de conditionnement pour récipients (3, 3') à usage pharmaceutique selon la revendication précédente, **caractérisée en ce que** ledit système (13) pouvant supporter les récipients (3, 3') comporte une partie en appui (14) contenue dans l'espace cylindrique (15) compris entre les générateurs cylindriques desdits trous (12) et est configuré afin de libérer un plan diamétral (16) dudit espace cylindrique (15).

9. Structure de conditionnement pour récipients (3, 3') à usage pharmaceutique selon la revendication précédente, **caractérisée en ce que** les plans diamétraux (16) sans espaces cylindriques (15), correspondant aux trous (12) de chaque rangée de trous (12), sont mutuellement coplanaires.

10. Structure de conditionnement pour récipients (3, 3') à usage pharmaceutique selon la revendication précédente, **caractérisée en ce qu'**aux plans diamétraux libres et coplanaires (16) est associé un volume total libre, afin de permettre l'introduction par en dessous d'un élément redressé (17) pour une action simultanée sur un ou plusieurs récipients (3, 3').

11. Structure de conditionnement pour récipients (3, 3') à usage pharmaceutique selon une ou plusieurs des revendications précédentes, **caractérisée en ce que** ledit élément de fermeture (5) est une feuille exfoliative du plateau (1).

12. Structure de conditionnement pour récipients (3, 3') à usage pharmaceutique selon une ou plusieurs des revendications précédentes, **caractérisée en ce que** ledit élément de fermeture (5) est un matériau sélectif perméable à un processus de stérilisation des contenus du plateau (1).

13. Structure de conditionnement pour récipients (3, 3') à usage pharmaceutique selon une ou plusieurs des revendications précédentes, **caractérisée en ce qu'**elle comporte un logement de protection fermé (19) qui garantit la stérilité du plateau (1) ou un logement comportant au moins une porte de transfert rapide pour son introduction dans une zone contrôlée.

14. Structure de conditionnement pour récipients (3, 3') à usage pharmaceutique selon une ou plusieurs des revendications précédentes, **caractérisée en ce qu'**au plateau (1) soit associé un élément d'identification et de traçabilité (18).

15. Utilisation d'une structure de conditionnement pour récipients (3, 3') à usage pharmaceutique selon une ou plusieurs des revendications précédentes dans une machine de traitement pour le déplacement automatique et/ou le remplissage automatique et/ou pour la fermeture automatique des récipients (3, 3').
